# EUROPEAN PATENT SPECIFICATION

(11) **EP 1 090 127 B1**
(45) Date of publication and mention of the grant of the patent: **28.05.2008**
(21) Application number: 99933376.8
(22) Date of filing: 23.06.1999
(51) Int. Cl.: C12N 15/53, C12N 9/04, C12Q 1/68, C12Q 1/25, A61K 38/44, A61K 48/00, A61K 31/07

(54) **ADH7 NUCLEOTIDES**
ADH7-NUKLEOTIDE
NUCLEOTIDES ADH7

(30) Priority: 26.06.1998 SE 9802294; 26.06.1998 US 90925 P
(43) Date of publication of application: 11.04.2001
(73) Proprietor: AstraZeneca AB, 151 85 Södertälje (SE)
(72) Inventor: OLSON, Lars, S-181 43 Lidingö (SE); BÜRVENICH, Silvia, S-182 53 Danderyd (SE); SYDOW, Olof, S-167 56 Bromma (SE); ANVRET, Maria, S-175 47 Järfälla (SE); ZHANG, Zhiping, S-141 33 Huddinge (SE)
(86) International application number: PCT/SE1999/001136
(87) International publication number: WO 2000/000621

(56) References cited:
- GB-A- 2 292 522
- US-A- 5 514 825
- BIOCHEMICAL AND BIOPHYSICAL RESEARCH COMMUNICATIONS. HIROKAZU YOKOYAMA ET AL: 'Upstream Structure of Human ADH7 Gene and the Organ Distribution of its Expression.' vol. 216, no. 1, 1995,, pages 216 - 222, XP002919874
- THE JOURNAL OF BIOLOGICAL CHEMISTRY MIRNA ZGOMBIC-KNIGHT: 'Genomic Structure and Expression of the ADH7 Gene Encoding Human Class IV Alcohol Dehydrogenase, the Form Most Efficient for Retinol Metabolism in Vitro.' vol. 270, no. 9, March 1995,, pages 4305 - 4311, XP002919875
- PROC NATL ACAD SCI SAMAD TA ET AL: 'Regulation of dopaminergic pathways by retinoids:activation of the D2 receptor promoter by members of the retinoic acid receptor-retinoid X receptor family' vol. 94, no. 26, December 1997,, pages 14349 - 14354, XP002919876
- MOLECULAR ENDOCRINOLOGY ROLF H. ZETTERSTRÖM ET AL: 'Retinoid X Receptor Heterodimerization and Developmental Expression Distinguish the Orphan Nuclear Receptors NGFI-B,Nurr1,and Nor1' vol. 10, no. 12, 1996,, pages 1656 - 1666, XP002919877
- PROC. NATL. ACAD. SCI. PETER MCCAFFERY: 'High levels of a retinoic acid-generating dehydrogenase in the meso-telencephalic dopamine system' vol. 91, August 1994,, pages 7772 - 7776, XP002919878
- DATABASE EMBL 5 May 1998 (1998-05-05), SAGE-ONO, K.: "Pharbitis nil PnC401 mRNA for leaf protein, comple cds." XPD85101
- DATABASE EMBL 5 May 1998 (1998-05-05), SAGE-ONO, K.: "Pharbitis nil PnC401 mRNA for leaf protein, comple cds."

## Description

### Technical field

The present invention relates to isolated human nucleic acids implicated in Parkinson's disease and the uses thereof, such as in diagnostic and prognostic methods and in pharmaceutical preparations.

### Background

Parkinson's disease is a neurodegenerative disease that strikes men and women alike. It is estimated that it affects about 15 out of 10.000 individuals and usually it first appears at an age of about 55-60 years. Despite considerable efforts, the reason for the symptom-causing degeneration of the midbrain dopamine neurons in patients afflicted by this crippling, non-curable disorder is not known. Moreover, while many of the symtoms of Parkinson's disease can be mimicked by lesions or neurotoxin administration to experimental animals, the disease itself is not known in any other species than man, thus hampering research aimed at understanding the etiology and the development of new therapies.

Exposure to toxic compounds in the environment remains one hypothetical cause of the disease although epidemiological studies have generated few clues as to its etiology. More recently, an interest in familial forms of the disease has indicated the presence of genetic components and linkage to an area on chromosome 4 has been reported and is discussed in more detail below.

Thus, in recent linkage studies, autosomal dominant or autosomal recessive types of Parkinson's disease have been mapped to several different loci in the human genome. Although these types of Parkinson's disease often differ from sporadic Parkinson's disease and constitute only a small fraction of the total patient population, the reported loci might confer susceptibility also for idiopatic forms of Parkinson's disease.

Polymeropoulos et al (Science 274, p.1197 (1996)) mapped autosomal dominant Parkinson to chromosome 4q21-q23. They called this area "PARK1". Later, they found a mutation in the gene for alpha-synuclein which segregated with the disease in one large Italian and three Greek kindreds and which they could not find in healthy control individuals. Synuclein was found to be contained abundantly in Lewy bodies, thus providing evidence of a causal link between mutation and disease. However, inspite of large efforts by other groups to find synuclein mutations in their own patients, they failed to identify any mutations in the alpha-synuclein gene in their material.

Interestingly, Vaughan et al. (Hum. Mol. Genet. 7, 751 (1998)) have reported one German family with autosomal dominant Parkinsonian to show linkage to the PARK1 region 4q21-q23. In this family, no mutations in alpha-synuclein could be identified. Said authors suggest that there might be another gene in the same locus that might account for the disease. In this case, the synuclein mutation in the Italian and Greek kindreds may be a marker that segregates with the "true" disease gene.

Accordingly, the prior art has not identified any gene responsible for idiopathic Parkinson's disease in humans. Thus, in the state of the art, there are still no reliable diagnostic and prognostic tools in this regard and thus, individuals at risk of developing Parkinson's disease have no safe way of knowing this beforehand. In addition, and maybe more importantly, patients suffering from Parkinson's disease cannot yet trust the science to provide them with a safe remedy of this highly unpleasant and incapacitating disease.

### Summary of the invention

The object of the present invention is to fulfill the need defined above in regard of Parkinson's disease. Accordingly, the present invention conclusively relates to the establishment of the identity of a human gene wherein Parkinson's disease is developped as well as to the specific mutations of said gene associated with the disease. More specifically, the human gene encoding alcohol dehydrogenase 7 (ADH7) has according to the present invention for the first time been shown to be significant in association with the development of Parkinson's disease by exhibiting one or more herein defined mutations triggering the disease. Accordingly, the novel findings according to the present invention also implicates a novel use of ADH7 in the diagnosis, treatment and/or prevention of Parkinson's disease. The invention will be disclosed in more detail below together with various advantageous novel uses.

### Brief description of the drawings

Figure 1 shows the previously published sequence of ADH7 (Zgombic *et al., supra).*
Figure 2 shows the GenBank records U16286 through 16293 harboring the human ADH7 gene sequences for the promoter, all exons and intronic sequences in a larger extend than displayed in figure 1.
Figure 3 shows Table 1, wherein the primers used to amplify eight fragments of the human ADH7 gene according to the section "Experimental" below are defined.
Figure 4 shows Table 2, wherein the sequences of the eight amplified mutated fragments M1-M7 according to the invention are compared with corresponding wildtype sections of the sequence disclosed in Figure 1 or the GeneBank entries according to Figure 2.
Figure 5 shows the localizations of the ADH7 gene mutations M1-M7 according to the invention.
Figure 6A shows Table 3, wherein A1 allele frequency is disclosed and compared for different groups, while Figure 6B shows Table 4 displaying the occurence of homozygotes for A1 and A3 among Parkinson patients.

### Detailed description of the invention

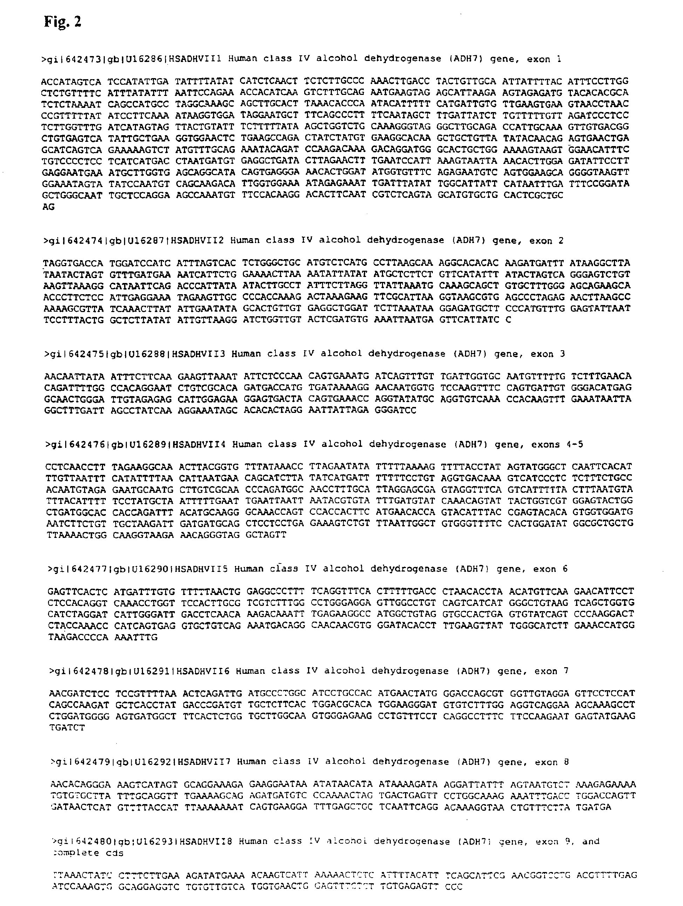

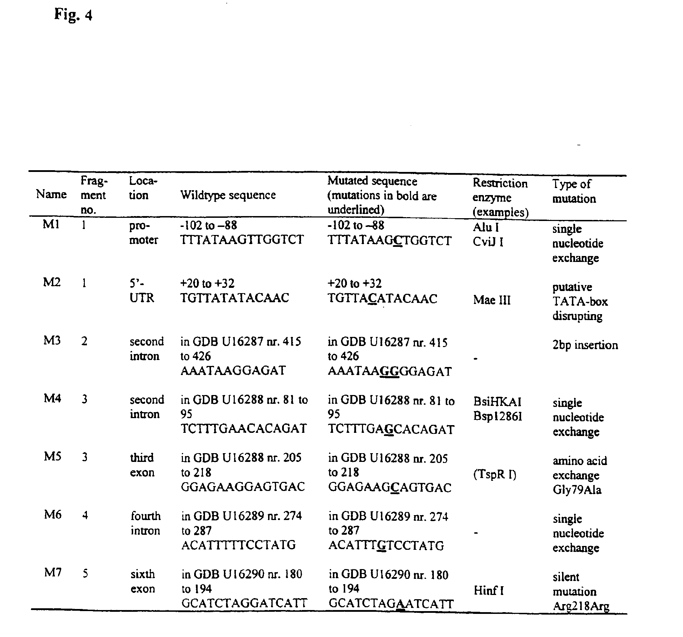

Thus, more specifically, in a first aspect, the present invention relates to the use of an alcohol dehydrogenase 7 (ADH7) associated nucleic acid, which comprises parts or all of the nucleotide sequence of the human wildtype ADH7 encoding gene, or a variant thereof, including at least one mutation selected from the group consisting of M2, M3, and M6 as defined in Figure 4. When interpreting the table of Figure 4 of the present application, reference is made to the GeneBank sequences disclosed in Figure 2. This is due to the fact that the ADH7 sequence published by Zgombic-Knight *et al. (supra)* in 1995 is not the complete sequence, but has later been complemented by the GeneBank references disclosed in Figure 2 and referred to in said table. Thus, as no established numbering of the totality of the hitherto sequenced sections exists, the present application uses both of these figures in order to identify the mutations according to the invention. It is to be understood that the present invention relates to the mutations M1-M7 as defined herein as well as to the same mutations defined by any alternative system of numbering, that is, to any functionally equivalent mutation irrespective of the denotation chosen. In conclusion, the present system of identifying the mutations M1-M7 according to the invention enables the skilled in this field to locate the mutations today as well as in any complete ADH7 sequence in the future. Further, the present invention will also enable the establishment of locations for mutations in related genes, such as ADH5.

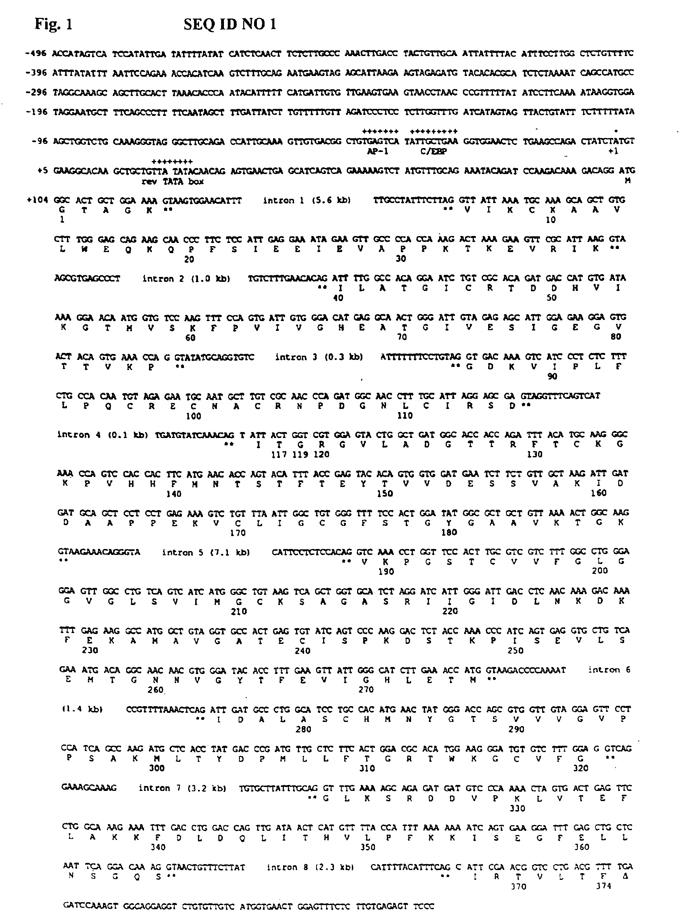

The present invention relates to a mutation selected from the group consisting of M2, M3, and M6 as such as well as to specific uses of any mutation selected from the group consisting of M2, M3, and M6. This is due to the fact that M 1 was actually published before the priority date of the present application (Zgombic-Knight et al, The Journal of Biological Chemistry, 1995, vol. 270, Issue of March 3, pp. 4305-4311). However, said publication described M1 within the therein presented wild type sequence, which has now been shown by the present inventors to be erraneous, as M1 is in fact a mutation associated with Parkinson's disease. Thus, at the previous publication of the base sequence of M1, the herein disclosed various advantageous uses thereof could not be foreseen, and are accordingly patentable aspects of the present invention. Further, the present invention also relates to the subsequence of the wild type ADH7 sequence corresponding to M1 *per se,* as said sequence has never been described before the present invention. In specific embodiments, the present invention relates to isolated nucleotides comprised of DNA sequences including at least one mutation selected from the group consisted of M2, M3, and M6, or the wild type sequence corresponding to M1, and at least about 10 bases of the adjacent and/or surrounding sequences shown in Figure 4 and also defined by SEQ. ID. NOS. 2, 3 and 6 and SEQ. ID. NO. 1, respectively. Said sequences may, in particular embodiments, be surrounded by, or adjacent to, any number of consecutive bases from the wild type human sequence defined in Figure 1, up to essentially all of said sequence. For use as a probe, a length of about 10 bases may be suitable in some methods, but the skilled in this field can easly choose a suitable length depending on the intended use. In one particular embodiment, the invention relates to an isolated nucleic acid encoding ADH7, which nucleic acid differs from the previously published sequence by including the correct wild type instead of the M1 mutation. The present ADH7 encoding sequence may be genomic or comprised of cDNA.

In one particular embodiment of this aspect of the invention, said mutation is the mutation denoted M2, which is in a putative TATA-box. Alternatively, the nucleotide according to the invention comprises any one of the mutations M3, or M6 or any combination thereof.

As mentioned above, the present inventors have for the first time identified the human gene encoding alcohol dehydrogenase 7 (ADH7) as a gene significant in association with Parkinson's disease in humans. According to the invention, a subset of patients suffering from Parkinson's disease exhibit one or more herein defined mutations, which thus trigger the disease. Accordingly, in a particular embodiment of the present invention, the nucleotide according to the invention is an isolated mutant ADH7 encoding gene capable of triggering Parkinson's disease in a human. Additionally or alternatively, the isolated mutant gene according to the invention is capable of passing on the disease to a later generation. In the present context, it is to be understood that the expression Parkinson's disease should be interpreted to include all kinds of parkinsonism. It is also to be understood that familial as well as non-familial forms of Parkinson's disease are included in said expression.

In the present application, the term "nucleic acid" refers to a deoxyribonucleotide or ribonucleotide polymer in either single- or double-stranded form, and encompasses known analogs of natural nucleotides that can function in a similar manner as naturally occurring nucleotides. When reference is made to the gene, it is to be understood that both DNA and cDNA are encompassed. -

Preferably, the present nucleotides comprise one or two of the above defined mutations surrounded by, or adjacent to, the human ADH7 sequence as disclosed in Figure 1 and 2 or essential parts thereof. In alternative embodiments, the present invention is not restricted to nucleotides corresponding to the complete human ADH7 gene or essential parts thereof, such as functional fragments thereof, or to analogues thereof, but is limited only by the presence of one or more of the herein defined mutations as well as to any fragment(s) surrounding the mutation(s). How long the surrounding fragments are will be determined by the intended future use thereof, as discussed below, but may be anywhere in a range from about 0-50, or about 50-100, preferably up to about 300, such as 200-300, and most preferably up to 1000 base pairs of the ADH7. Thus, the present invention encompasses the above defined mutations in an ADH7 environment. In one embodiment of the present invention, the sequence of the ADH7 is essentially as defined in Figure 1 and 2, that is, at least one mutation is flanked by a sequence encoding the human enzyme ADH7. It is to be understood that all variants of the ADH7 nucleotide sequence disclosed herein are also within the scope of the present invention.

The present invention also relates to a nucleotide which, under stringent conditions, is capable of specific hybridisation to any one of the nucleotides according to the invention and defined above. In the present context, the term "hybridising specifically to" refers to the binding, duplexing or hybridising of a molecule only to a particular nucleotide sequence under stringent conditions when that sequence is present in a complex mixture of DNA or RNA. In the present context, the term "stringent conditions" refers to conditions, under which a probe will hybridise to its target sequence, but to no other sequences. Stringent conditions are sequence-dependent and will be different in different circumstances. The one skilled in this field will easily choose the suitable conditions in the present context Generally, stringent conditions are selected to be about 5°C lower than the thermal melting point (Tm) for the specific sequence at a defined ionic strength and pH. Typically, stringent conditions will be those in which the salt concentration is less than about 1.0 M Na ion, such as about 0.01-1.0 M, at a pH of about 7.0-8.3 and the temperature is between about 30°C and 60°C, depending on the length of the nucleotide. Stringent conditions may also be achieved by the addition of destabilizing agents, such as formamide. Such a nucleotide according to the invention may be of any length in accordance with the above defined.

Thus, the present invention also relates to the use of any of the present nucleotides as probes. As used herein, the term "nucleic acid probe" is defined as a nucleic acid capable of binding to a target nucleic acid of complementary sequence through one or more types of chemical bonds, usually by complementary base pairing, usually through hydrogen bond formation. The probes according to the invention may include natural (i.e. A, G, C or T) or modified bases (such as 7-deazaguanosine, inosine etc.). The probes according to the invention may be joined by other linkages than phosphodiester bonds, such as peptide bonds if the probe is a peptide nucleic acid (PNA), which is also an aspect of the invention, as long as the hybridisation is not interfered with. The probes according to the invention are preferably directly labeled, e.g. with isotopes, chromophores, lumiphores, chromogens etc, or indirectly labeled, such as with biotin to which a streptavidin complex may later bind. By assessing the presence or absence of the probe, the presence or absence of the selected sequence or subsequence, preferably one or more of the mutations according to the invention, may be detected. Such probes are preferably used in the diagnosis of Parkinson's disease, such as in the kits according to the invention, discussed in more detail below.

The present nucleotides may also be used as primers, e.g. for PCR, which for example may be present in a kit. Further, as discussed below, they may be used to generate immunogenic polypeptides or fusion proteins for use in generating specific antibodies which recognise the mutant epitope.

The nucleotides according to the invention are cloned, or amplified, by any *in vitro* or *in vivo* methods, such as the polymerase chain reaction (PCR), the ligase chain reaction (LCR), the transcription-based amplification system (TAS), the self-sustained sequence replication system (SSR) or cell based cloning and amplification, wherein the cell may be any suitable cell, such as a bacterium, a cultured cell line etc. A wide variety of cloning and amplification methods are well known to the skilled in this field, see e.g. Sambrook et al., (1989); Molecular Cloning: A Laboratory Manual, 2nd Ed, Vol. 1-3).

The invention also relates to peptides, polypeptides or proteins specifically synthesized to bind to any one of the present nucleotides, such as the mutations and/or its surrounding/adjacent sequences. In the present context, the terms "peptide", "polypeptide" and "protein" are used interchangeably to refer to a polymer of amino acid residues. The terms "peptide", "polypeptide" and "protein" apply to amino acid polymers, in which one or more amino acid residues are artificial chemical analogues of a corresponding naturally occurring amino acid, as well as to naturally occurring amino acid polymers. In a specific embodiment, the invention relates to the present peptide for use as a medicament.

The peptides, polypeptides and proteins according to the invention may be synthesized using standard chemical peptide synthesis techniques well known to the skilled person. For solid phase synthesis, see e.g. Barany and Merrifield, Solid-Phase Peptide Synthesis, in The Peptides: Analysis, Synthesis, Biology, vol 2: *Special Methods in Peptide Synthesis.* The present peptides and polypeptides may also be produced using recombinant DNA technology. Generally, this involves creating a DNA sequence that encodes the desired peptide, e.g. as a recombinant sequence encoding the present peptide as part of a fusion protein, placing the DNA in an expression cassette under the control of a particular promoter, expressing the peptide in a host and isolating the expressed peptide. In case of a protein, the renaturation thereof may be required. The nucleic acids encoding the peptides or proteins may be expressed in a variety of host cells, including E.coli. other bacterial hosts, yeast, and various higher eucaryotic cells, such as COS, CHO and HeLa cell lines and myeloma cell lines. The gene will be operably linked to appropriate expression control sequences for each host. For E.coli, this includes a promoter such as the T7, trp or lambda promoters, a ribosome binding site and preferably a transcription termination signal. For eucaryotic cells, the control sequences will

The expression of a mutated ADH7 gene may be detected and/or quantified by detecting or quantifying the expressed peptide encoded by the nucleotide according to the invention. A number of means well known to those of skill in the art are available. These may include analytic biochemical methods, such as electrophoresis, capillary electrophoresis, high performance liquid chromatography (HPLC), thin layer chromatography (TLC), hyperdiffusion chromatography and the like, or various immunological methods, such as fluid or gel precipiting reactions, immunodiffusion (single or double), immunoelectrophoresis, radioimmunoassay (RIA), enzyme-linked immunosorbent assays (ELISAs), immunofluorescent assays, Western blotting etc.

In the present application, the term "ADH7 analogue" is to be construed as comprising any other alcohol dehydrogenases than ADH7. "antibody" refers to a polypeptide substantially encoded by an immunoglobulin gene or fragments thereof, which specifically binds to and recognize an analyte (antigen). The recognized immunoglobulin genes include the kappa, lambda, alpha, gamma, delta, epsilon and mu constant region genes as well as the myriad of immunoglobulin variable regions. The antibodies according to the invention are raised to any peptide or polypeptide encoded by one of the present mutations, optionally together with flanking ADH7 regions. The antibodies are preferably monoclonal, but may also be polyclonal. Monoclonal antibodies are prepared from cells secreting the desired antibody. The antibodies are then screened for binding to normal or modified polypeptides or screened for agonistic or antagonistic activity, e.g. activity mediated through a peptide according to the invention. Specific monoclonal antibodies, monoclonal and polyclonal, will usually bind with a KD of at least about 0.1mM, more usually at least about 50 µM and most preferably at least about 1 µM or better. Methods of producing antibodies are known to those of skill in this area, see e.g. Coligan (1991) Current Protocols in Immunology, Wiley Greene, NY; Harlow and Lane (1989) Antibodies: A Laboratory Manual, Cold Spring Harbor Press, NY and Goding (1986) Monoclonal Antibodies: Principles and Practice (2d ed.) Academic Press, New York, NY.

In some instances, it is desirable to prepare monoclonal antibodies from various mammalian hosts, such as mice, rodents, primates, humans etc. Description of techniques for preparing such monoclonal antibodies are found in e.g. Stites et al. (eds.) Basic and Clinical Immunology (4th ed), Lange Medical Publications, Los Altos, CA, and references cited therein. Summarized briefly, this method proceeds by injecting an animal with an immunogen. The animal is then sacrified and cells are taken from its spleen, which are fused with myeloma cells. The result is a hybrid cell or a "hybridoma", that is capable of reproducing *in vitro.* The population of hybridomas is then screened to isolate individual clones, each of which secrete a single antibody species to the immunogen. In this manner, the individual antibody species obtained are the products of immortalized and cloned single B cells from the immune animal generated in response to a specific site recognized on the immunogenic substance. Alternative methods of immortalization include transformation with Epstein-Barr virus, oncogenes, retroviruses etc. The present invention relates to chimeric antibodies as well as to humanised antibodies.

In a further aspect, the present invention relates to the use of a nucleotide, antibody or polypeptide according to the invention in the prognostic and/or diagnostic detection of Parkinson's disease associated mutations in patients or individuals with a Parkinson predisposition as well as to kits for performing such diagnosis. Thus, according to the invention, a defective ADH7 gene may be detected, implicating an increased risk of developing Parkinson's disease or alternatively establishing the diagnosis of the disease. Such a kit may comprise one or more of the nucleotides according to the invention as reagents. Alternatively, the kit according to the invention will comprise means for detecting at least one of the mutations herein denoted M1-M7, said means being e g. primers, restriction enzymes etc. The kit will preferably also include instructions for the use thereof. Thus, the ADH7 gene or gene product may be detected using an amplification based assay. In an amplification based assay, all or part of the gene or transcript (e.g. mRNA or cDNA) is amplified and the amplification product is then detected. Amplification-based assays are well known to those of skill in the art and disclosures are easily found in the literature. The mutated sequences provided by the present invention are sufficient to enable one of skill to routinely select primers to amplify any portion of the ADH7 gene.

The present invention also relates to screening methods. All of the mutations in the nucleotide sequence of the gene coding for human ADH7 can be detected by either of the following methods (a-j), which are described in detail in: (1) Mutation Detection, A Practical Approach, R.G.H. Cotton, E. Edkins and S. Forrest (editors), The Practical Approach Series, Oxford University press (1998) and (2) Finding Mutations, The Basics, J.R. Hawkins, Oxford University Press (1997).
(a) Single strand conformation polymorphism analysis (SSCA, also called SSCP), alone or in combination with heteroduplex analysis (HA) is one of the most widely used approaches for mutation detection. (b) Denaturing gradient gel electrophoresis (DGGE) is the name of a whole family of similar methods that are based on the reduction in electrophoretic mobility of a DNA molecule in a dense medium during denaturation. (c) The ribonuclease protection assay or RNase cleavage assay (RPA), the chemical cleavage of mismatch (CMM) assay and mutation detection using T4 endonuclease VII (EMC) are effective methods to detect DNA-DNA or DNA-RNA mismatches caused by mutations. (d) Hybridisation with sequence specific oligonucleotide probes (SSOP) takes advantage of the fact that under stringent conditions, a single basepair mismatch can prevent hybridisation of short complementary oligonucleotide probes. (e) Ligation assays such as the oligonucleotide ligation assay (OLA) comprise further ways of detecting changes in DNA sequences. (f) Direct sequencing was the method used according to the present invention when detecting the seven different mutations (M1-M7) in the human ADH7 gene, and it constitutes one of the most sensitive methods of mutation detection. (g) A rather new method originating from direct sequencing is called minisequencing or solid phase minisequencing. (h) Selective amplication of specific alleles (PASA, also called ASA or ASP) makes it possible to detect mutations already during amplification of the fragment of interest by PCR. (i) With the protein truncation test (PTT), one can identify mutations that induce stop codons in DNA sequence after translation of the sequence into protein. (j) Restriction fragment length polymorphism (RFLP) analysis makes use of endonucleases which recognize and cleave specific nucleotide sequences. Mutations in DNA can either change the sequence so that an endonuclease which cuts the wildtype sequence cannot recognize and cut the mutated sequence, or it can induce a new cleavage site for an endonulease not cutting the wildtype sequence. In figure 4, examples are given for naturally occurring endonucleases that can be used in order to detect the mutations M1-M7. In sequences that cannot be distinguished by naturally occurring enzymes, mutations can be analyzed by primer-introduced restriction analysis, a method which alters the sequence surrounding the mutation.

Thus, the present invention also relates to a method of screening, wherein at least one mutation selected from the group consisting of M2, M3 and M6 as defined in Figure 4 of the present application is detected in order to diagnose Parkinson's disease. In one embodiment, a screening method according to the invention uses restriction enzymes specifically recognizing a specific nucleotide sequence surrounding any one of the mutations M2, M3, and M6, as defined in Figure 4 in presence or absence of the respective mutation in the diagnosis of Parkinson's disease. In an alternative embodiment, biological or chemical agents detecting mismatches of nucleic acids are used in order to detect any of the mutations M2, M3, and M6 as defined in Figure 4 in the diagnosis of Parkinson's disease.

More specifically, amplification assays and hybridisation probes according to the invention may be used to specifically target abnormalities in the ADH7 gene, which according to the present invention has been identified as a gene triggering Parkinson's disease. A variety of solid-phase detection techniques can be used, see e.g. Fodor et al. (1991) Science, 251:767-777; Sheldon et al. (1993) Clinical Chemistry 39(4): 718-719, and Koza et al. (1996) Nature Medicine 2(7): 753-759. See also Tjissen (1993): Laboratory Techniques In biochemistry And Molecular Biology - Hybridisation with nucleic acid probes, parts I and II, Elsevier, New York.

In addition, the invention also relates to the use of isolated human wildtype ADH7, or any molecule which is capable of taking over a missing function of ADH7, or of compensating for a lack of ADH7 function or abolishing an ADH7 dysfunction, in the manufacture of a therapy or medicament for treating Parkinson's disease, preferably Parkinson's disease caused by one or more of the above defined mutations in the ADH7 gene. In an alternative embodiment, the invention relates to the use of an alcohol or retinoid normally metabolized by ADH7, or any alternative ADH7 substitute, such as an enzyme, in the manufacture of a therapy or medicament for treating Parkinson's disease, preferably Parkinson's disease caused by one or more of the above defined mutations in the ADH7 gene. Thus, the invention also relates to a pharmaceutical composition for treating and/or preventing Parkinson's disease caused by one or more of the herein described mutations in the ADH7 gene. The composition comprises wildtype ADH7 in a therapeutically effective dose and a pharmaceutically acceptable carrier, such as an aqueous carrier, e.g. buffered saline and the like, which is sterile and free of undesirable matter. In an alternative embodiment of this aspect of the invention, the pharmaceutical composition comprises a substrate or a product of ADH7, such as an alcohol or retinoid normally metabolized by ADH7, or any alternative ADH7 substitute, such as an enzyme, in a therapeutically effective dose and a pharmaceutically acceptable carrier. Even though the use of retinoid compounds have been suggested before for the prevention and treatment of conditions and diseases associated with human papilloma virus (see US 5 514 825), the present invention suggests for the first time such uses for the treatment or prevention of Parkinson's disease. The amounts effective of wildtype ADH7 as the active ingredient will depend upon the severity of the condition and the general state of the patient's health. A composition according to the invention may also comprise suitable excipients and auxiliary substances as required, pH-adjusting and buffering agents, toxicity adjusting agents, stabilizers, etc., such as conventionally used in the pharmaceutical industry. The present composition may be administered in a variety of unit dosage forms depending on the method of administration. For example, unit dosage forms suitable for oral administration include powder, tablets, pills, capsules and lozenges, the oral preparation being most preferred for reasons of simplicity. Actual methods for preparing parenterally administrable compositions are known or apparent to those skilled in the art and are described in more detail in such publications as Remington's Pharmaceutical Science, 15th ed., Mack Publishing Company, Easton, Pennsylvania (1980).

The present invention also relates to all of the pharmaceutical aspects discussed above, wherein the previously disclosed peptides, polypeptides or proteins capable of binding a nucleotide comprising the sequence of at least one mutation selected from the group consisting of, M2, M3, and M6 are used.

The pharmaceutical use of ADH7 according to the invention is based on the following proposed pathways.

### 1. Direct involvement of ADH7 in retinoid metabolism

A retinoid-handling aldehyde dehydrogenase was recently shown to be specifically expressed in the midbrain dopamine neurons of the rat brain. To convert retinal to retinoic acid, one alcohol dehydrogenase and one aldehyde dehydrogenase are needed. The dopamine neurons also express the transcription factor Nurr1 and recent knock-out experiments demonstrate that this factor is required for the development of dopamine neurons as well as for proper function of dopamine neurons in postnatal adult life. Nurr1 may itself be activated by retinoid, which however remains to be proven. Importantly, Nurr1 forms heterodimers with RXR, which is activated by 9-cis retinoic acid. Thus, a mechanism is proposed, in which the dopamine neurons are critically dependent upon a specific combination of an aldehyde and an alcohol dehydrogenase (ADH7) needed to generate retinoids necessary to activate RXR-Nurrl mediated transcriptional control of vital genes in these neurons.

### 2. Involvement of ADH7 in dopamine metabolism

To metabolize the transmitter dopamine, aldehyde and alcohol dehydrogenases are needed. It is proposed that ADH7 serves this role, malfunction leading to toxic accumulation of metabolites and thereby damaging the dopamine neurons.

### 3. ADH7 as a detoxifying enzyme in the gastrointestinal tract

ADH7 can also convert aldehydes into less aggressive alcohols, thus protecting from the absorption of aldehydes in food, or generated during digestion. It is proposed that if ADH7 is defect, then toxic aldehydes reach the circulation and pass the blood-brain barrier to damage dopamine neurons. Alternatively, such aldehydes lead to secondary effects, eventually damaging the dopamine neurons.

Accordingly, there are several important implications of the mutations detected according to the present invention as well as the herein for the first time recognized role of ADH7 and/or related enzymes.

Firstly, as mentioned above, the present invention may be utilized in novel diagnostic tools. Before the present invention, there was been no way of predicting or diagnosing Parkinson's disease prior to the onset of the symtoms. When symtoms occur, patients have typically already lost a very large number, in reality, the majority, of their dopamine nerve cells. The early dignosis which the present invention enables together with preventive measures will be most valuable and fill a need that has been felt for a long time by both physicians and patients. Diagnostic tools, such as assays, e.g. for screening of samples, or similar methods, wherein the nucleotides according to the present invention are used, also enable a subclassing of the disease with a prognostic value and will assist in the differential diagnosis towards a series of Parkinson-like diseases and so called Parkinson+ cases. Simple blood tests will suffice to this end.

Secondly, the present invention will provide a base for future development of novel therapies. Since ADH7 is proposed to be directly involved in the disease process, the now recognized mechanism of ADH7 according to the invention will form an important basis for the development of novel therapeutica and drugs. One such novel drug is a pharmaceutical preparation comprising one or more retinoid agonists together with a suitable pharmaceutically acceptable carrier. Another such drug is one that can handle toxic aldehydes as well as one that can compensate for the lack of ADH7 as a dopamine metabolizing enzyme. This may e.g. be in the form of a pill, that acts in the intestinal tract, or any other suitable form as discussed above. Thus, the present invention also relates to a method of treating a patient by administering a drug that compensates for the lack of ADH7, e.g. by administering a therapeutically effective dose of ADH7.

Thirdly, the present invention also relates to animal models of Parkinson's disease. As mentioned above, Parkinson's disease does not exist in animals, which hitherto has imposed a substantial problem in the research within this field. Research have therefore been based on models in which those neurons that die in the human disease are damaged mechanically or chemically in animals to generate similar symtoms. As the ADH7 mutations according to the invention can cause Parkinson's disease in human beings, the teaching according to the invention now for the first time enables the generation of "Parkinson mice" using gene targeting techniques. For example, based on the present invention, once the ADH7 corresponding genes have been identified in mouse, a classical knock-out mouse model may be produced. Accordingly, the present invention also relates to genetically manipulated animals, such as mice, that contain one or more of the mutations according to the invention. In order to produce such an animal, a nucleotide according to the invention is introduced in a suitable vector by standard protocols. (For production of transgenic animals, such as mice, see US patent 5 455 169 in the name of Mullen, and references cited therein.) Thus, in the animals according to the invention, the human genomic defect(s) can be precisely replicated, leading to animals that develop a disease with the human characteristics. The model animals according to the invention, preferably model mice, will be of great value to researchers and the pharmaceutical industry alike as tools for the development of new treatments and therapies, such as medicaments.

The present invention also relates to methods for diagnosis, prevention or treatment of Parkinson's disease. A method of detecting the presence a mutation according to the invention may for example include the steps of obtaining a biological sample from a human subject, which sample is analyzed by isolating DNA from said sample, digesting said DNA with a restriction enzyme cleaving at suitable sites, and analyzing a restriction pattern from said digestion in order to identify said mutation. Methods for detecting mutations in DNA are e.g. discussed by Landegren, U, GATA 9, 1992, pp 3-8.

### Detailed description of the drawings

Figure 1 shows the nucleotide sequences of exons, small parts of the introns at the exon-intron borders and the promoter for the human class IV ADH7 gene as previously published (Zgombic-Knight, et al. (1995), Journal of Biological Chemistry, vol. 270, p. 4305). The nucleotide sequences for all nine exons are shown as well as some sequence upstream and downstream of each. The location of the eight introns are indicated with their approximative sizes in parentheses. The 5' and 3' ends of each intron contain the conserved GT/AG splice site sequences indicated with asterisks. The transcription initiation site is shown at position +1 at an adenine labeled with a closed circle. Upstream of the transcription start site, two potential transcription factor binding sites in the promoter (AP-1 and C/EBP) are indicated based upon consensus sequence matches. Downstream of the transcription start site, a TATA box in the reverse orientation (rev TATA box) is shown. The predicted amino acid sequence of the class IV ADH7 coding region (downstream of the initiator methionine at position +101) is numbered according to the homology with class I ADH, which is used for numbering all vertebrate ADH sequences (Jörnvall *et al.,* 1987). The region downstream of the initiator methionine is actually 373 amino acids in length, one shorter than class I ADH due to the apparent deletion of codon 118, which is noticed when the sequences of all classes of human ADH are aligned (Satre *et al.,* 1994). The stop codon is indicated by a triangle and 64 bp of the 3'-untranslated region are shown.

Figure 2 shows the GenBank records U16286 through 16293 harboring the human ADH7 gene sequences for the promoter, all exons and intronic sequences in a larger extend than displayed in figure 1. The nucleotides are grouped in blocks of 10 nucleotides in order to facilitate determination of the positions of the mutated nucleotides described in the following and referred to in Figure 4. The sequence as displayed in figure 2 can be accessed via the internet at the address: http://www.ncbi.nhn.nih.gov/htbin-post/Entrez/query?uid=642480&form=6&db= n&Dopt=f.

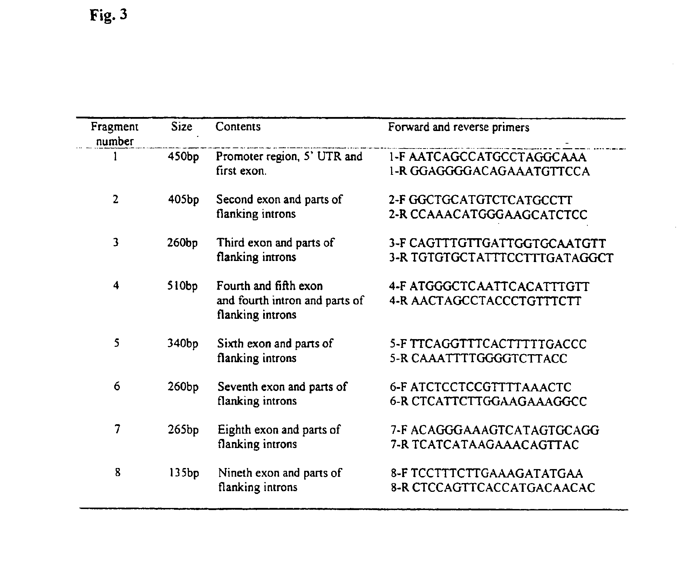

Figure 3 shows Table 1, wherein the primers used to amplify eight fragments of the human ADH7 gene are shown, which will be described in further detail in the experimental part below.

Figure 4 shows Table 2, wherein sequence fragments present within the said eight amplified fragments according to the invention are compared with the published ADH7 sequence of Figure 1. The nucleotide numbers refer to Figure 1 and nucleotides that do not appear, or are not numbered, therein are referred to by their positions in the GenBank database entries according to Figure 2. The mutated nucleotides are in bold and underlined. Examples of restriction enzymes useful in order to detect the presence or absence of the mutations are given for those mutations where such enzymes are known. TspRI has been found by the inventors not to be suitable for detection of the mutation M5 in spite of its theoretical ability to do so. Whether this depended on the experimental conditions or a mistake in the literature reporting the cutting abilities of this enzyme was not decided.

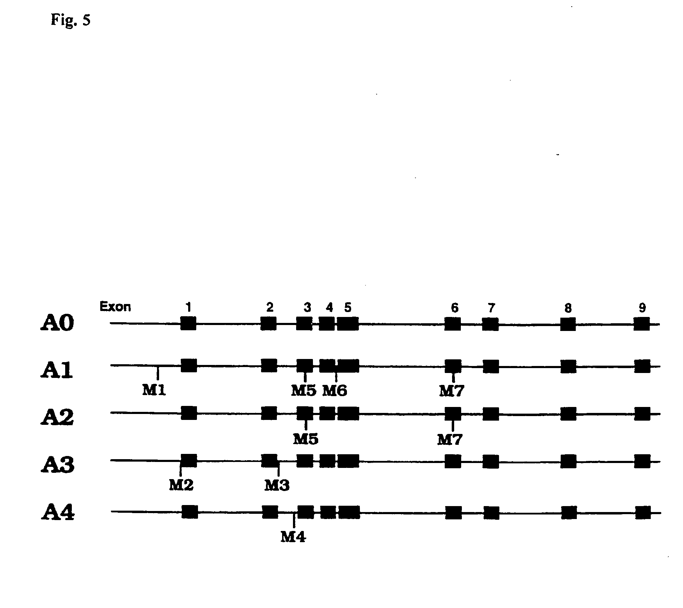

Figure 5 shows the distribution of the seven mutations (M1 to M7) on the five different alleles (A0-A4) identified by the present inventors. The wildtype allele (A0) is the most frequent allele in the Swedish control population. By definition, it does not contain any mutations.

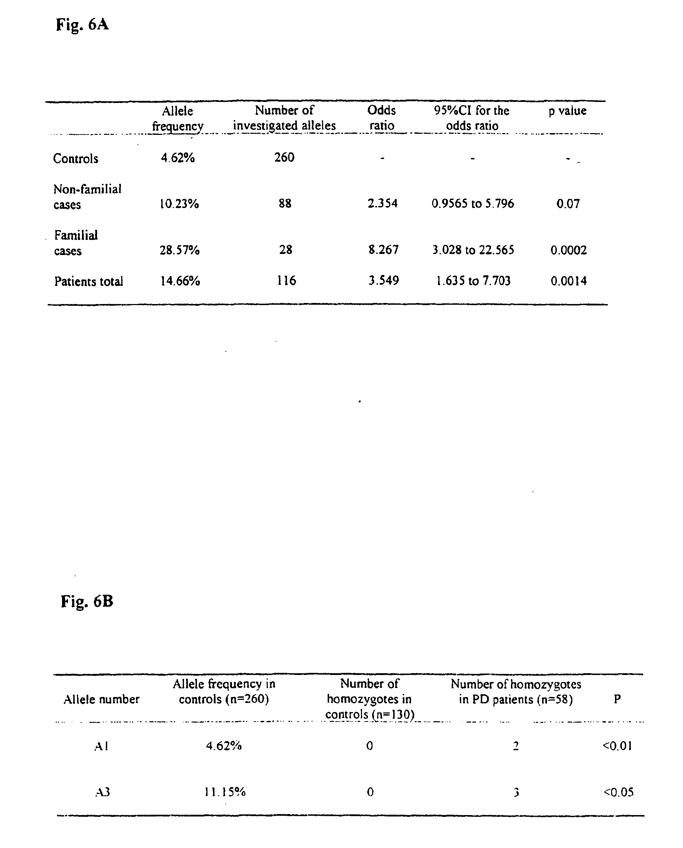

Figure 6A shows the allele frequencies of the A1 allele in Parkinson patients and controls. The A1 allele frequencies were determined by direct sequencing of fragment 1 in patients and controls and checking for the presence of M1 which is unique for A1. Fisher's exact test was performed on all differences between patients and controls and the differences between the total patient population and controls and the patient subgroup of familial cases and controls was found to be highly significant. The odds ratios and 95% confidence intervals for the odds ratios are also shown for all differences between frequencies in controls and patient populations. All p-values are two-tailed p-values. This highly significant association of the A1 allele with Parkinson's disease suggests a role for the A1 allele in the pathogenesis of the disease.

Figure 6B documents the finding of two homozygotes for the A1 allele and three homozygotes for the A3 allele among the 58 Parkinson patients while none of the 130 healthy controls was found to be homozygous for any of these alleles.

The present inventors assumed a binominal distribution of all alleles in the population and took the likelihood for a person to get two copies of one of the mutated alleles as the likelihood for "success" in order to determine whether one would expect to find 2 or 3 homozygotes for A1 or A3, respectively, among the patients. Given the allele frequencies displayed in the second column in the control population, the chance to find two (or more) homozygotes for A1 among 58 individuals is very low (P<0.01). The chance to find three (or more) homozygotes for A3 is also low (P<0.05). That means that the chance to find both two homozygotes for A1 and three homozygotes for A3 in the same sample of 58 individuals is extremely low (P<0.0005). This accumulation of homozygotes for the mutated alleles A1 and A3 further strongly suggests involvement of ADH7 in the pathogenesis of Parkinson's disease.

### EXPERIMENTAL

The present experimental disclosure is only presented as illustrating the invention as defined by the claims supported by the description and is in no way intended to limit the scope of the invention.

### General procedure

Basically, the present invention results from an interest in the role of retinoic acid in the nigrostriatal dopamine system. Dopamine neurons contain all the necessary components for retinoic acid mediated transcriptional control via the nuclear receptors RAR (binding all-trans retinoic acid) and RXR (binding 9-cis retinoic acid) as well as for Nurr1 transcription, presumably activated by another retinoid. The enzyme aldehyde dehydrogenase 1 (Aldh1, which previously was denoted Ahd2), previously shown to metabolize retinaldehyde to retinoic acid, is strongly and specifically expressed in dopamine neurons. Retinaldehyde is generated from retinol by an alcohol dehydrogenase and the alcohol dehydrogenase isozyme that has been shown to be most potent for this conversion *in vitro* is denoted ADH7 in humans, belonging to the class IV of the alcohol dehydrogenases.

Further, the present inventors observed that the gene for this enzyme lies within the alcohol dehydrogenase cluster on chromosome 4q21-25, which is overlapping with the PARK1 mapping area (4q21-23). ADH7 has been mapped to chromosome 4q23-34, an area partially overlapping the PARK1 area. Furthermore, this area overlaps the chromosomal region with the highest lod scores in the German family showing linkage to this region without having mutations in the gene for alpha-synuclein. Thus, according to the present invention, it was presumed that the gene for ADH7 would be a very strong candidate gene for mutations in cases of Parkinson's disease.

In order to verify this, the very sensitive method of direct sequencing was chosen in order to look for mutations in the coding and promoter region of the ADH7 gene. Sequences of the promoter, all exons and parts of the introns at the exon-intron boundraries have been published by Zgombic-Knight et al (The Journal of Biological Chemistry, 1995, vol. 270: "Genomic Structure and Expression of the ADH7 Gene Encoding Human Class IV Alcohol Dehydrogenase, the Form Most Efficient for Retinol Metabolism in Vitro"*).*

Firstly, ADH7 was sequenced in ten Parkinson patients with confirmed family history of Parkinson's disease. Primers used to amplify eight fragments are shown in Table 1 (Figure 3). Four alleles containing a total of seven alterations from the published ADH7 sequence (Figures 1 and 2) were identified. Allele A1 (A1) contains four changes from the wildtype sequence at four different locations, A2 and A3 two changes at two different loci and A3 one change.

The distribution of the seven mutations (M1-M7) on four different alleles (A1-A4) is shown in Figure 5.

Allele A1 contains two single nucleotide exchanges, one of which is located in the promoter and the other in the fourth intron (M1 and M6). Additionally, there is one sequence alteration in exon 3 (M5) and one in exon 6 (M7, see figure 4). While the base exchange in exon 6 is a silent mutation (Arg218Arg), the exchange in exon 3 leads to a Gly79A1a substitution. A2 contains both of the exonic mutations present in A1 (M5 and M7) but lacks both the mutation in the promoter (M1) and the mutation in the fourth intron (M6). In allele A3, a double base pair insertion in the second intron (M3) and a single nucleotide exchange in the 5' untranslated region (M2) were observed. The change in the 5' untranslated region is located in a TATA-like element located 22 bases downstream of the transcription site and in reversed direction. Because the ADH7 gene lacks a normal TATA-box 20 bases upstream of the transcription initiation site, and no other sequence elements normally found in TATA-less promoters are present in its promoter, this TATA-like element is proposed to be involved in transcription initiation. Functionality of a TATA-like element located around 20 bases downstream of the transcription initiation site in reversed direction has been shown previously.

Allele 3 contains one single base substitution (M4) located in the second intron, 6 bases upstreams from the intron/exon border.

Next the allele frequencies of A1 to A4 was determined in 58 Parkinson patients and 130 healthy controls from the same geographical area. Fourteen of the patients had a first or second degree relative with confirmed or very probable Parkinson's disease. They were classified as "familial Parkinson patients". All alleles were present in both controls and patients. Allele 1 containing the Gly79Ala exchange was found to be significantly more frequent in patients than in controls (figure 6A). The difference was most prominent in patients with family histories of the disease.

Moreover, two patients with familial background were found to be homozygous for the mutation (figure 6B). This was an unlikely event (P<0.01) as the frequency for homozygosity calculated from the allele frequencies was only 1 in 468 individuals. There was no significant difference of A2 frequencies between patients (3.45%; n=116) and controls (6.25%; N=256) and we did not find any homozygotes for A2 among all groups. Allele A3 was also found to be equally frequent in controls and the total Parkinson patient population (11.14% and 10.34% respectively). The distribution of A3 among the Parkinson patients was however uneven with frequencies of 5.68% (n=88) of the alleles in non-familial PD and 25% (n=28) in familial PD. The difference between controls and familial PD was not found to be significant (p=0.063). However, this significance may have been masked by the high frequency of A1 in the familial cases. When removing the A1 alleles from both controls and patients, the difference in A3 frequencies between controls and patients also became significant (odds ratio=4.07; 95%CI = 1.5 to 11.03; p<0.01). Three individuals among the 58 patients were homozygous for A3, two of them having family history of the disease and one being uninformative in regard to affected relatives. The expected occurrence of homozygosity for the A3 allele is 1 in 76 individuals, which means that the occurrence of three or more homozygotes in 58 individuals is significantly different from the expected numbers (P<0.05). No compound heterozygotes for A1 and A3 was found among the patients. The frequencies of A4 were not significantly different between controls (5.21%; n=96) and patients (2.59 %; n=116). No homozygotes for A4 were found in any of the groups.

Thus, the results suggest that there is an association of two alleles (A1 and A3) at the ADH7 locus with Parkinson's disease. Five homozygous individuals among the 58 investigated patients for either A1 (n=2) or A3 (n=3) are reported. Assuming a binominal distribution of the alleles in the population and taking the frequency of homozygosity as the likelihood for success, the probability for the homozygous cases to occur by chance is very low. The probability of finding two homozygotes for A1 (P<0.01) together with three homozygotes for A3 (P<0.05) is even more unlikely (P<0.0005). The fact that both events occurred in the same material therefore further suggests correlation to disease. In our cases with family history, A1 and A3 together accounted for more than half (53.5%) of all alleles present.

### Mutation screening

DNA was extracted from blood samples of 58 consecutive Parkinson outpatients according to standard protocols. All patients fullfilled recognized diagnostic criteria for Parkinson's disease. Fourteen of the patients had one or more first- or second-degree relatives with confirmed or very probable Parkinson's disease. Among the remaining 44 sporadic patients, ten reported other cases of tremor in their families.

All patients were informed about the aim of the study and the study was approved by the Swedish ethical committee (filed under Dnr. 96-377).

The primers shown in Table 1 (Figure 3) were used to amplify eight fragments of the human ADH7 gene containing the coding and 5'regulatory regions.

Polymerase chain reaction (PCR) was carried out according to standard protocols using Taq DNA polymerase (SIGMA). 35 cycles were run with 94°C for 40 seconds, 56°C for 45 seconds and 72°C for one minute.

After PCR, the samples were run on 1% low melting agarose gels and visualized using UV-translumination after ethidiumbromide staining. The gel pieces containing the amplified fragments were then melted and DNA was extracted (PCR preps DNA purification kit, SDS) according to the manufacturer's instructions.

After purification from the agarose gel pieces, the DNA fragments were sequenced (Thermo Sequenase radiolabeled terminator cycle sequencing kit, Amersham) following the manufacturer's instructions. The reaction products were run on 6% acrylamid sequencing gels (National Diagnostics). Film (Sterling Diagnostic Imaging) was put on the gels for autoradiography.

When comparing the sequence of the eight amplified fragments with the published sequence (Zgombic-Knight M, *supra,* genebank accession nr. U16286-U16293), which is disclosed in Figures 1 and 2 of the present application, the alterations disclosed in Table 2 (Figure 4) were found (nucleotide numbers refer to Figure 1, nucleotides that do not appear, or are not numbered, therein are referred to by their positions in the GenBank database entries displayed in Figure 2).

## Claims

1. Use of a nucleic acid sequence including at least one mutation selected from the group consisting of the ADH7-mutations M2, M3 and M6, as defined in SEQ. ID. NOS. 2, 3 and 6, respectively and at least 10 bases of the adjacent and/or surrounding sequences shown in Fig. 4, in the diagnosis of Parkinson's disease.

2. The use according to claim 1, wherein said ADH7-mutation is M2 (SEQ. ID. NO. 2).

## Patentansprüche

1. Verwendung einer Nukleinsäuresequenz, die wenigstens eine Mutation, ausgewählt aus der Gruppe der ADH7-Mutationen M2, M3 bzw. M6, wie sie in SEQ. ID. NO. 2, 3 bzw. 6 definiert sind, sowie wenigstens 10 Basen der benachbarten und/oder umgebenden Sequenzen, dargestellt in Fig. 4, enthält, bei der Diagnose von Morbus Parkinson.

2. Verwendung nach Anspruch 1, wobei es sich bei der ADH7-Mutation um M2 (SEQ. ID. NO. 2) handelt.

## Revendications

1. Utilisation d'une séquence d'acide nucléique comprenant au moins une mutation choisie dans le groupe constitué des mutations de ADH7 M2, M3 et M6, telles que définies respectivement dans les SEQ ID NO : 2, 3 et 6, et au moins 10 bases des séquences adjacentes et/ou environnantes présentées en figure 4, pour le diagnostic de la maladie de Parkinson.

2. Utilisation selon la revendication 1, selon laquelle ladite mutation de ADH7 est M2 (SEQ ID NO : 2).


### SEQUENCE LISTING

<110> KAROLINSKA INNOVATIONS AB
<120> ADH7 NUCLEOTIDES
<130> 55828
<140>
   <141>
<160> 7
<170> PatentIn Ver. 2.1
<210> 1
   <211> 15
   <212> DNA
   <213> HUMAN
<400> 1
   tttataagtt ggtct 15
<210> 2
   <211> 13
   <212> DNA
   <213> HUMAN
<220>
   <221> mutation
   <222> (6)
<400> 2
   tgttacatac aac 13
<210> 3
   <211> 14
   <212> DNA
   <213> HUMAN
<220>
   <221> mutation
   <222> (7)..(8)
<400> 3
   aaataagggg agat 14
<210> 4
   <211> 15
   <212> DNA
   <213> HUMAN
<220>
   <221> mutation
   <222> (8)
<400> 4
   tctttgagca cagat 15
<210> 5
   <211> 14
   <212> DNA
   <213> HUMAN
<220>
   <221> mutation
   <222> (8)
<400> 5
   ggagaagcag tgac 14
<210> 6
   <211> 14
   <212> DNA
   <213> HUMAN
<220>
   <221> mutation
   <222> (7)
<400> 6
   acatttgtcc tatg 14
<210> 7
   <211> 15
   <212> DNA
   <213> HUMAN
<220>
   <221 > mutation
   <222> (9)
<400> 7
   gcatctagaa tcatt 15